# EUROPEAN PATENT APPLICATION

(11) **EP 1 230 952 A2**
(43) Date of publication of application: **14.08.2002**
(21) Application number: 02250159.7
(22) Date of filing: 10.01.2002
(51) Int. Cl.: A61P 17/00, A61K 31/565

(54) **Combinations of estrogen and progestogen for preventing and treating skin aging**

(30) Priority: 16.01.2001 US 261765 P; 16.08.2001 US 312961 P
(71) Applicant: WARNER-LAMBERT COMPANY, Morris Plains New Jersey 07950 (US)
(72) Inventor: Brandman, Jane, c/o Pfizer Global Res. & Develop., Ann Arbor, Michigan 48105 (US); Hanley, Rochelle M., c/o Pfizer Global Res. & Dev., Ann Arbor, Michigan 48105 (US)
(74) Representative: Simpson, Alison Elizabeth Fraser

(57) **Abstract**

Skin aging is prevented and treated with a composition comprising an estrogen and a progestin.

## Description

### FIELD OF THE INVENTION

This invention relates to a method for preventing and treating skin aging in a mammal by administering compositions containing a progestin and an estrogen. This invention also relates to a method for preventing and treating acne by administering compositions containing a progestin and an estrogen.

### BACKGROUND OF THE INVENTION

The population of the world is currently undergoing a significant incidence of aging which is leading to disease states of the skin and a problematic overall aging process. Some of the problems associated with aging are a change in skin coloration, quality, and texture. This is broadly referred to as skin aging, which is characterized by loss of elasticity, the occurrence of wrinkling and dryness, and the development of irregular pigmentation. Because there are medical and psychological problems associated with skin aging, our increasing aging population is at risk for complications associated with this disease in great proportions. This condition is exasperated by global warming which leads to diminished skin hydration and the erosion of the ozone layer which exposes the population to elements that cause skin photoaging.

The life expectancy of women has increased and predictions for the year 2050 estimate the average age at 81 years. Thus, women are at great risk of experiencing the afflictions of the skin associated with aging, especially photoaging (Bergfeld, *Int. J. Fertil*., 1999;44(2):83-95). There is a need for effective diagnosis, management, delay of the onset, and treatment of skin aging for the maintenance of good health and improving the overall quality of life as the normal aging process develops.

Skin aging is characterized by a decline in skin collagen and skin thinning which is associated with a delay in wound healing and the formation of hemorrhagic skin patches. Other clinical manifestations of skin aging include the slowing of metabolic processes, skin dryness, increased wrinkling of the skin, decreased vascularization of the skin, reduced skin elasticity and reduced quality of skin appearance such as mottle pigmentation, a condition of spotting with patches of color. Skin aging is also associated with increased rosiness, increase in pore size of the skin, dryness, and skin roughness.

Deterioration of skin condition with age results from a combination of factors. These include genetic, chronologic aging, photoaging, behavioral aging, catabolic aging and gravitational aging and environmental factors. Another type of skin aging is endocrine aging which is determined by dysfunction or aging of hormonal systems such as the ovaries of females. Skin aging is a particularly debilitating for women reaching menopause.

The clinical sequelae of cutaneous aging may result in aging of the overall appearance. This in turn can have a negative impact on various aspects of quality of life issues including social interactions, occupational functioning, and the psychological state of the individual.

Providing methods for maintaining healthy skin and for treating and delaying skin aging is of significant clinical importance as the life expectancy of the population increases.

There are few effective treatments available for managing skin aging. Tretinoin emolliement cream is used for the treatment of photoaging. Alpha-hydroxy acids, antioxidants, antiandrogens, moisturizers, and exfoliants are also used to manage skin aging symptoms. Surgical procedures are also used such as dermabrasion, chemical peels, soft tissue augmentation, laser resurfacing, botulism toxin (Bergfeld, *Supra*., 1999).

Skin disease is common with skin aging, and a typical manifestation of skin disease is acne. Acne is often one of the afflictions of the skin that women, in particular, experience. The incidence of acne affects more that 17 million people in the United States.

Acne vulgaris and the more severe cystic acne are common forms of acne. Acne vulgaris is characterized by an eruption of the skin, most often occurring on the back or chest. Such eruptions consist of comedones also known as whiteheads and blackheads. Papules are raised areas and pustules which have an inflammatory base. Cystic acne is defined as "acne with the formation of cysts enclosing a mixture of keratin and sebum in varying proportions," Dorland's Illustrated Medical Dictionary, 27^{th} ed. Philadelphia: W.B. Saunders Co., hereinafter referred to as "Dorland's".

Patients can experience acne with symptoms such as comedones and inflammatory lesions (papules or pustules), nodules, red and raised lesions (papules, pustules and nodules).

Acne can have a negative impact on the physical appearance and condition of the skin. The disfigurement caused by acne progresses with the aging process. When acne heals, the contracture can cause permanent changes in the skin, such as scarring and hyperpigmentation and keratosis, which are characteristics of aging. The effect over time accumulates with physical effects such as pitting and discoloration occurring.

Acne has a negative impact on the patient's well being, affecting social interactions, psychological health, quality of life, self esteem and confidence. Acne can exasperate existing psychological disorders such as depression. Furthermore, a patient's economic opportunities can be adversely affected by the negative impact to physical and mental health caused by acne.

An object of this invention is to provide a method for treating symptoms of skin aging and to delay detrimental effects of skin aging.

An object of this invention is to provide a novel method for treating skin aging comprising administering an effective amount of compositions containing an estrogen in combination with a progestin.

A further object of this invention is to provide a method for treating skin aging comprising administering an effective amount of compositions containing a fixed quantity of a synthetic estrogen in combination with a synthetic progestin.

Still another object of this invention is to provide a method for treating symptoms of acne and to delay detrimental effects of acne comprising administering an effective amount of compositions containing an estrogen in combination with a progestin.

A further object of this invention is to provide a method for treating acne comprising administering an effective amount of compositions containing a synthetic estrogen in combination with a synthetic progestin.

### SUMMARY OF THE INVENTION

This invention provides a method for delaying the onset and treating skin aging in a patient in need of treatment comprising administering an effective amount of compositions containing an estrogen and a progestin.

In a preferred embodiment, the invention provides a method for delaying the onset and treating skin aging in a patient in need of treatment comprising administering an effective amount of a composition containing a fixed quantity of an estrogen in combination with a progestin.

In a more preferred embodiment, the invention provides a method for delaying the onset and treating skin aging in a patient in need of treatment comprising administering an effective amount of a composition containing a fixed quantity of norethindrone acetate (NA) and ethinyl estradiol (EE).

In a still further preferred embodiment, the invention is a method for delaying the onset and treating skin aging in a patient in need of treatment comprising administering an effective amount of a composition containing 1 mg NA and 5 µg EE in a pharmaceutically acceptable carrier; and

A preferred method for delaying the onset and treating skin aging in a patient in need of treatment comprising administering an effective amount of 1 mg NA and 10 µg EE in a pharmaceutically acceptable carrier.

Another preferred embodiment of this invention provides a method for treating patients in need of delaying the onset and treating skin aging comprising administering an effective amount of compositions containing an estrogen and a progestin. This invention provides a method for treating patients in need of delaying the onset and treating skin aging using a composition which comprises administration of a progestin while cyclically administering an estrogen as described by United States Patent No. Re. 36,247 which is hereby incorporated by reference. This invention also contemplates administering the wide variety of doses of progestin and estrogen described by United States Patent No. Re. 36,247 which is incorporated by reference.

Yet another preferred aspect of this invention also provides a method for treating patients in need of fixed quantity of a synthetic estrogen in combination with a synthetic progestin. This invention also contemplates administering estrogen/progestogen combinations as described in United States Patent No. 5,010,070, 5,552,394 or 5,898,032 which are hereby incorporated by reference. This invention involves administering progesterone according to the doses described in United States Patent No. 5,208,225 and United States Patent No. Re. 36,247 while administering estrogen in doses cited in United States Patent No. 5,208,225, more specifically where the estrogenic agent is a fixed quantity of NA. Preferably, this invention discloses the administration of 0.1 to 1.0 mg of NA wherein the ratio of NA to progesterone or EE is about 50:1 to about 200:1. This invention more specifically provides a method for preventing and treating skin aging in a patient in need of treatment comprising administering an effective amount of compositions containing a fixed quantity of NA and EE. In a preferred embodiment this invention provides a method for preventing and treating skin aging in a patient in need of treatment comprising administering an effective amount compositions containing of 1 mg NA and 5 µg EE. A preferred embodiment of this invention provides a method for preventing and treating skin aging in a patient in need of treatment comprising administering an effective amount of 1 mg NA and 10 µg EE in a pharmaceutically acceptable carrier.

A preferred aspect of this invention provides a method for preventing and treating acne in a patient in need of treatment comprising administering an effective amount of compositions containing an estrogen and a progestin.

In a preferred embodiment, this invention provides a method for preventing and treating acne comprising administrating an effective amount of compositions containing an estrogen and progestin as described in United States Patent No. 5,010,070.

In a more preferred embodiment, the invention provides a method for preventing and treating acne in a patient comprising administering an effective amount of a composition containing NA and EE.

An especially preferred method for preventing and treating acne in a patient comprises administering an effective amount of Estrostep® (Warner-Lambert Company) which contains 1 mg NA and a gradually increasing dose of EE; 20 mcg × 5 days, 30 mcg × 7 days, and 35 mcg × 9 days in a pharmaceutically acceptable carrier.

Estrostep **Fe®** provides for a continuous dosage regimen consisting of 21 oral contraceptive tablets and seven ferrous fumarate tablets. The ferrous fumarate tablets are present to facilitate ease of drug administration via a 28-day regimen, are nonhormonal, and do not serve any therapeutic purpose.

Women taking hormone replacement therapy experience adverse events such as breakthrough bleeding and/or spotting. The occurrence of breakthrough bleeding and/or spotting causes many patients to discontinue hormone replacement therapy.

femhrt™ (Warner-Lambert Company) is a formulation and regimen with superior bleeding and/or spotting control. The femhrt formulation decreases the incidence of unwanted vaginal bleeding and/or spotting. The improved profile may be due to the dose and type of progestin used in the femhrt formulation. Women taking femhrt are able to attain amenorrhea and are more likely to continue their prescribed dosage regimen, experience less physician visits, and experience cost savings.

A preferred aspect of this invention is drawn to a method for treating acne using estrogen and progestin as described in United States Patent No. 5,552,394.

### DETAILED DESCRIPTION OF THE INVENTION

The compounds required to practice the method of this invention are estrogens and progestins in combination. Compositions containing an estrogen and a progestin to be administered are described in United States Patent No. 5,208,225 and United States Patent No. Re. 36,247. These compounds are useful in delaying the onset and treating skin aging.

To practice this invention, an estrogen and a progestin can be prepared as pharmaceutical compositions suitable for oral or parenteral administration, as well as transdermal and intranasal dosing. In a preferred embodiment, progestin and estrogen compositions are prepared according to the procedures described in United States Patent No. 5,208,225 and United States Patent No. Re. 36,247. Preferred formulations of progestin and estrogen compositions are combined with common excipients and carriers for oral administration in the form of tablets, capsules, syrups, solutions, cachets, buccal seals as well as other excipients. Preferred excipients for a progestin-estrogen combination tablet composition for oral administration is calcium stearate, lactose monohydrate, microcrystalline cellulose, and cornstarch. The composition can also be prepared as a parenteral mixture for injection, for example intramuscularly, intracutaneously, subcutaneously, intraduodenally, or intraperitoneally. The compound can be dissolved in an suitable diluent. This compound can be administered by inhalation, for instance intranasally.

The following definitions apply to the terms used in this Specification and in the claims.

"femhrt™" is a pharmaceutical composition commonly in the form of a white D-shaped tablet which contains 1 mg NA [(17-alpha)-17-(acetyloxy)-19-norpregna-4-en-20-yn-3-one] and 5 mcg EE [(17-alpha)-19-norpregna-1,3,5(10)-trien-20-yn-2,17-diol]. Each tablet also contains calcium stearate, lactose monohydrate, microcrystalline cellulose, and cornstarch.

The structural formulas are as follows:

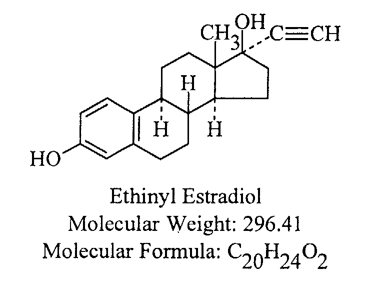

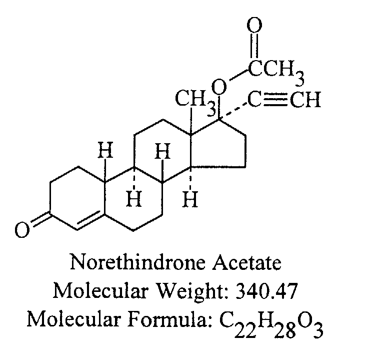

Such a formulation with properties as described above is commonly known as, femhrt having 1 mg NA/5 mcg EE or femhrt containing 1 mg NA/10 mcg EE and is a continuous dosage regimen of a progestin-estrogen combination for oral administration.

femhrt 1 mg NA/5 mcg EE and 1 mg NA/10 mcg EE is recommended to be given once daily for delay of the onset and for the treatment of mild to severe skin aging symptoms associated with menopause.

femhrt 1 mg NA/5 mcg EE pill is usually prescribed to be taken once a day at about the same time each day. If a dose is missed, the missed dose should be taken as soon as the patient remembers. If it is almost time for the next dose, the patient is instructed to skip the missed dose and take only the next regularly scheduled dose. The patient is advised not to take two doses at the same time.

The preferred treatment contemplated in this invention is, a combination tablet containing NA and EE. Each tablet contains either 1 mg NA and 10 µg EE, or 1 mg NA and 5 µg EE.

The compounds required to practice the method of this invention are estrogens and progestins in combination. Compositions containing an estrogen and a progestin to be administered are described in United States Patent No. 5,208,225 and United States Patent No. Re. 36,247 and United States Patent No. 5,010,070. These compounds are useful in preventing and treating acne.

The term "patient" means a mammal, particularly a human, having skin aging or showing symptoms associated with skin aging. A patient can also be an animal such as a dog, cat, horse, or cow.

The term "delay the onset of skin aging" means to slow or otherwise avert the occurrence of symptoms associated with skin aging.

The term "prevent acne" means to slow or otherwise avert the occurrence of symptoms associated with acne.

The term "treating skin aging" means managing the treatment of the patient to control the symptoms associated with skin aging.

The term "treating acne" means managing the treatment of the patient to control the symptoms associated with acne.

The term "acne" means "an inflammatory disease of the pilosebaceous unit...acne vulgaris is a chronic inflammatory disease of the pilosebaceous apparatus, the lesions occurring most frequently on the face, chest, and back. The inflamed glands may form small pink papules, which sometimes surround comedones so they have black centers, of form pustules or cysts..." (Dorland's). Acne vulgaris and the more severe cystic acne are forms of acne. Acne vulgaris is characterized by an eruption of the skin, most often occurring on the back or chest. Such eruptions consist of comedones also known as whiteheads and blackheads. Papules are raised areas and pustules which have an inflammatory base. Cystic acne is defined as "acne with the formation of cysts enclosing a mixture of keratin and sebum in varying proportions." (Dorland's). Patients can experience acne having comedones and inflammatory lesions (papules or pustules), nodules, red and raised lesions (papules, pustules, and nodules). When acne heals the contracture that occurs causes oftentimes permanent changes in the skin such as scarring and hyperpigmentation and keritosis. The effect over time accumulates with physical effects such as pitting and discoloration occurring. Nodules may be suppurative or hemorrhagic. Nodules may be evaluated as few, many, moderate, or several nodules.

The term "skin aging" means changes in the skin such as the slowing of metabolic processes. These symptoms include changes caused by chronologic aging which are determined by the passage of time which has a genetic component such as programmed atrophy of the dermis and subcutaneous tissue. Skin aging also is caused by the effects on the skin of the gravitational force, such as skin sagging. Endocrine aging is associated with hormonal changes which are associated with the dysfunction or aging of the hormonal system. Aging of the skin is associated with a decrease in skin vascularization which can cause the yellowing of the skin. Aging is associated with thinning of the dermis which can cause skin atrophy, a decrease of dermal cellularity effecting irregular texture, loss of fiber elasticity causing fine lines, and a deterioration of mechanical properties resulting in skin laxity. Aging is associated with skin changes such as the slowing of metabolic processes, thinning epidermis, a reduction in density of hair follicles, sweat ducts, and sebaceous glands. Aging is associated with the loss of blood vessels, dermal collagen, fat, and elastic fibers. Such symptoms of skin aging contribute to the gaunt appearance of true old age.

Behavioral aging is determined by such factors as diet, tobacco, alcoholic abuse, and drug use. Photoaging is determined by such factors as ultraviolet and infrared irradiations. Photoaging contributes to skin coarseness, lentigines, fine lines, telangiectasias, and solar keratoses. Skin aging can also be caused by exposure to wind and chemicals. In skin aging, cell maturation is altered effecting coarse texture and solar keratoses. Melanocyte alteration occurs causing solar lentigines and mottled pigmentation. Decreased collagen number and strength and solar elastosis results in fine wrinkling of the skin. Aging also causes loss of collagen support of vessels effecting senile purpura. Also, skin aging causes the alteration of the vascular network characterized by a yellow hue and loss of pink color of the skin. An increase in metabolic processes, a state of chronic inflammation of the skin, irregular pigmentation, elastosis (coarsening and yellow discoloration), roughness and dryness of the skin, and telangiectasia are also characteristic of extrinsic aging.

Catabolic aging is another type of aging determined by chronic intercurrent debilitation disease such as infections and cancers.

Genetic aging is determined by the patient's genetic composition and can result in premature aging and phototype aging.

Treatment of skin aging as contemplated herein means not only controlling the medical ravages brought on by the aging process, but includes the overall improving in the quality of life associated with the aging process. Treatment of skin aging as contemplated herein also includes improving the psychological and social well-being associated with manifestations of aging process.

This invention also contemplates improving the health of the patients skin.

The term "effective dose" means the amount of a progestin and an estrogen required to delay the onset or treat symptoms manifested by skin aging or acne in a particular patient. For example, norethindrone acetate is completely and rapidly deacetylated to norethindrone after oral administration, and the disposition of NA is indistinguishable from that of orally administered norethindrone. Norethindrone acetate and EE are rapidly absorbed from femhrt 1/5 tablets, with maximum plasma concentrations of norethindrone and EE generally occurring 1 to 2 hours postdose. Both are subject to first-pass metabolism after oral dosing, resulting in an absolute bioavailability of approximately 64% for norethindrone and 55% for EE. For example, a method for preventing and treating acne in a patient in need of treatment comprising administering an effective amount of Estrostep which contains 1 mg NA and a gradually increasing dose of ethinyl estradiol; 20 mcg × 5 days, 30 mcg × 7 days, and 35 mcg × 9 days in a pharmaceutically acceptable carrier. Other dosage forms may be administered.

"Estrogens" are well-known steroidal compounds. Numerous estrogens are known, and many have been used as hormonal replacements in women. Estrogens are largely responsible for the development and maintenance of the female reproductive system and secondary sex characteristics. Although circulating estrogens exist in a dynamic equilibrium of metabolic interconversions, estradiol is the principal intracellular human estrogen and is substantially more potent than estrone and estriol at the receptor level. The primary source of estrogen in normally cycling adult women is the ovarian follicle, which secretes 70 to 500 mcg of estradiol daily, depending on the phase of the menstrual cycle. After menopause, most endogenous estrogen is produced by conversion of androstenedione, secreted by the adrenal cortex, to estrone by peripheral tissues. Thus, estrone and the sulphate conjugated form, estrone sulphate, are the most abundant circulating estrogens in postmenopausal women. The pharmacologic effects of EE are similar to those of endogenous estrogens.

"Progestins" are also well-known steroidal compounds that are characterized in that they cause a biological response in animals similar to the natural hormone progesterone. Many synthetic progestins are known and have been used for hormone replacement and contraception. Progestins enhance cellular differentiation and generally oppose the actions of estrogens by decreasing estrogen receptor levels, increasing local metabolism of estrogens to less active metabolites, or inducing gene products that blunt cellular responses to estrogen. Progestins exert their effects in target cells by binding to specific progesterone receptors that interact with progesterone response elements in target genes. Progesterone receptors have been identified in the female reproductive tract, breast, pituitary, hypothalamus, bone, skeletal tissue and central nervous system. Progestins produce similar endometrial changes to those of the naturally occurring hormone progesterone.

The term "fixed combination of estrogenic and progestin agents" means a combination that gives relief from menopausal symptoms with minimal side effects. For example, such fixed combinations are compositions and methods in which a formulation containing a fixed estrogen/progestin ration to be administered to female individuals for conditions associated with hormone deficiency. A composition containing a fixed dosage of EE, for example 1.0 to 1.0 mg, yields, when administered in continuous sequence, acceptable hormone levels in patients. Fixed combinations of NA and EE, especially doses useful for low tablet forms where the ratio of NA may be from about 1:100 to about 1:1000 and EE from about 1:2000 to about 1:100,000, the final tablet weight. The process employs a two component drug dilution introduced onto tableting excipients.

### Pharmacokinetics

### Absorption and Bioavailability

Norethindrone acetate is completely and rapidly deacetylated to norethindrone after oral administration, and the disposition of NA is indistinguishable from that of orally administered norethindrone. Norethindrone acetate and EE are rapidly absorbed from femhrt 1/5 tablets, with maximum plasma concentrations of norethindrone and EE generally occurring 1 to 2 hours postdose. Both are subject to first-pass metabolism after oral dosing, resulting in an absolute bioavailability of approximately 64% for norethindrone and 55% for EE. Bioavailability of femhrt 1/5 tablets is similar to that from solution for norethindrone and slightly less for EE. Administration of NA/EE tablets with a high fat meal decreases rate but not extent of EE absorption. The extent of norethindrone absorption is increased by 27% following administration of NA/EE tablets with food.

The full pharmacokinetic profile of femhrt 1/5 (1 mg NA/5 mcg EE) was not characterized due to assay sensitivity limitations. However, the multiple-dose pharmacokinetics were studied at a dose of 1 mg NA/10 mcg EE in 18 postmenopausal women. Mean plasma concentrations are shown below (Figure 1) and pharmacokinetic parameters are found in Table 1. Based on a population pharmacokinetic analysis, mean steady-state concentrations of norethindrone for 1 mg NA/5 mcg EE and 1/10 are slightly more than proportional to dose when compared to 0.5 mg NA/2.5 mcg EE tablets. It can be explained by higher sex hormone binding globulin (SHBG) concentrations. Mean steady-state plasma concentrations of EE for the 0.5 mg NA/2.5 mcg EE tablets and femhrt 1/5 tablets are proportional to dose, but there is a less than proportional increase in steady-state concentrations for the NA/EE 1/10 tablet.

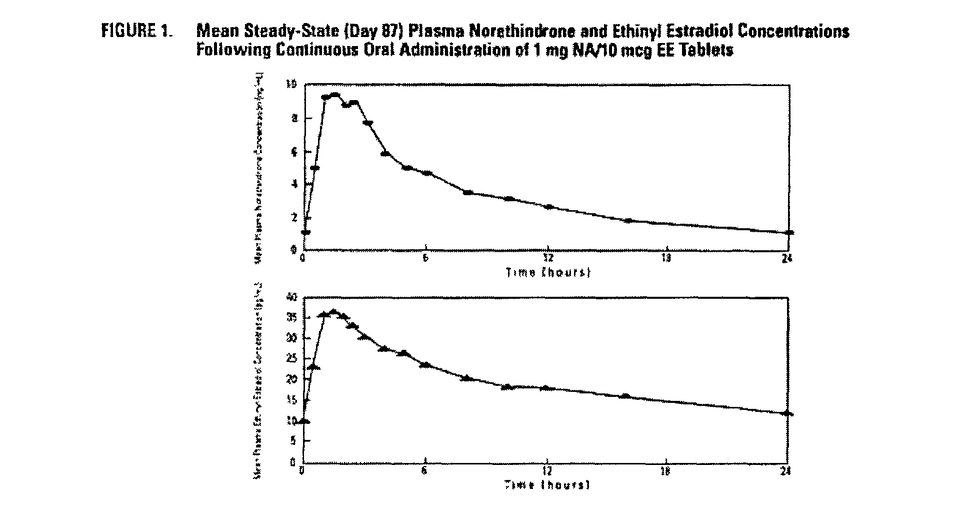

**TABLE 1**

| Mean (SD) Single-Dose (Day 1) and Steady-State (Day 87) Pharmacokinetic Parameters^{a} Following Administration of 1 mg NA/10 mcg EE Tablets | | | | | |
|---|---|---|---|---|---|
| | Cmax | Tmax | AUC(0-24) | CL/F | t½ |
| Norethindrone | ng/mL | Hr | ng·hr/mL | mL/min | Hr |
| Day 1 | 6.0 (3.3) | 1.8 (0.8) | 29.7 (16.5) | 588 (416) | 10.3 (3.7) |
| Day 87 | 10.7 (3.6) | 1.8 (0.8) | 81.8 (36.7) | 226 (139) | 13.3 (4.5) |
| Ethinyl Estradiol | pg/mL | Hr | pg·hr/mL | mL/min | Hr |
| Day 1 | 33.5 (13.7) | 2.2 (1.0) | 339 (113) | ND^{b} | ND^{b} |
| Day 87 | 38.3 (11.9) | 1.8 (0.7) | 471 (132) | 383 (119) | 23.9 (7.1) |

| | | | | | |
|---|---|---|---|---|---|
| ^{a} Cmax = Maximum plasma concentrations; tmax = time of Cmax; AUC(0-24) = Area under the plasma concentration-time curve over the dosing interval; CL/F = Apparent oral clearance; t½ = Elimination half-life. | | | | | |
| ^{b} ND = Not determined. | | | | | |

Based on a population pharmacokinetic analysis, average steady-state concentrations (Css) of norethindrone and EE for femhrt 1/5 (1 mg NA/5 mcg EE) are estimated to be 2.6 ng/mL and 11.4 pg/mL, respectively.

The pharmacokinetics of EE and NA were not affected by age (age range 40-62 years) in the postmenopausal population studied.

### Distribution

Volume of distribution of norethindrone and EE ranges from 2 to 4 L/kg. Plasma protein binding of both steroids is extensive (>95%); norethindrone binds to both albumin and SHBG, whereas EE binds only to albumin. Although EE does not bind to SHBG, it induces SHBG synthesis.

### Metabolism

Norethindrone undergoes extensive biotransformation, primarily via reduction, followed by sulfate and glucuronide conjugation. The majority of metabolites in the circulation are sulfates, with glucuronides accounting for most of the urinary metabolites. A small amount of NA is metabolically converted to EE, such that exposure to EE following administration of 1 mg of NA is equivalent to oral administration of 2.8 mcg EE. Ethinyl estradiol is also extensively metabolized, both by oxidation and by conjugation with sulfate and glucuronide. Sulfates are the major circulating conjugates of EE and glucuronides predominate in urine. The primary oxidative metabolite is 2-hydroxy EE, formed by the CYP3A4 isoform of cytochrome P450. Part of the first-pass metabolism of EE is believed to occur in gastrointestinal mucosa. Ethinyl estradiol may undergo enterohepatic circulation.

The following detailed clinical studies demonstrate that estrogen-progestin pharmaceutical compositions are effective in delaying the onset of skin aging and treating skin aging in patients in need of treatment.

### EXAMPLE 1

This example is a randomized, double-blind, double-dummy, placebo-controlled, multicenter study assessing the effects of NA and EE in the control of mild to moderate age-related skin changes in postmenopausal women.

This study investigates the ability of NA and EE to delay the onset and treatment of skin aging in postmenopausal women.

The object of this study is to assess the effects of femhrt™ (1 mg NA/5 µg EE) or 1 mg NA/10 µg EE compared to placebo in the control of skin wrinkling, dryness, and laxity associated with aging in postmenopausal women.

The study population consists of healthy postmenopausal women, aged 45 to 60 years, with skin changes associated with aging evaluated as mild to moderate.

This study is a 48-week randomized, double-blind, double-dummy, placebo-controlled, multicenter study.

Women will be randomized in a 1:1:1 ratio to receive either 1 mg NA/5 µg EE, 1 mg NA/10 µg EE, or placebo for 48 weeks.

The following measurements at Week 48 will be considered as primary efficacy parameters: wrinkling, laxity/sagging, texture/dryness, and wrinkle depth in the periorbital (crow's feet) area determined by image analysis of skin replicas using the area under the curve (Ra) examined in an east-west (EW) direction.

The secondary efficacy parameters include the above four primary efficacy measurements at Week 24. They also include the following at Weeks 24 and 48: Wrinkle depth in the jowl area determined by image analysis of skin replicas using shadows examined in a NS direction and maximum shadow width; wrinkle depth in the periorbital (crow's feet) area as determined by Ra in a north-south (NS) direction and shadows examined in both a NS and EW direction; skin elasticity of the jowl and upper cheek areas will be determined by timed deformation and recoil of the following specific areas:
- Wrinkling of the periorbital (crow's feet) area;
- Wrinkling of the perioral area;
- Wrinkling of the suborbital area;
- Laxity/sagging of the suborbital area;
- Laxity/sagging of the jowl area;
- Texture/dryness of the forehead;
- Texture/dryness of the cheeks;
Clinical panel assessment of subject overall skin appearance using subject photographs at selected study centers; and also a Menopause-Specific Quality-of-Life questionnaire (MENQOL), including supplemental modules covering skin and hair specific issues, treatment satisfaction, and additional items not covered in the original MENQOL.

In the statistical rationale and analysis, the overall type 1 error rate will be determined to be 0.05. Since there are four primary objectives, the method developed by Hochberg will be utilized for the multiple comparison adjustments. Assume P1 ≤P2 ≤P3 ≤P4 are the ordered observed p-values:
Step 1: P4 will be compared with 0.05. If P4 ≤0.05, we can claim all 4 primary comparisons are statistically significant.
Step 2: If P4 >0.05, we then compare P3 with 0.05/2 = 0.025. If P3 ≤0.025, we can claim the comparisons associated with P1 to P3 are all statistically significant.
Step 3: If P3 >0.025, we compare P2 with 0.05/3 = 0.0167. If P2 ≤0.0167, we can claim the comparisons associated with P1 and P2 are statistically significant.
Step 4: If P2 >0.0167, we compare P1 with 0.05/4 = 0.0125. If P1 ≤0.0125, we claim the comparison associated with P1 is statistically significant.

Assuming that the higher dose group has higher efficacy, the step-down trend test will be performed, i.e., four primary comparisons between the 1/10 dose group and the placebo group will first be made. Only for those objectives that are significant will comparison between the 1/5 group and the placebo group then be made. These comparisons will be made at the same level as the Hochberg procedure on the higher dose. This closed procedure will insure the overall type 1 error to be controlled at 0.05.

If at least one primary comparison is significant, the study will be considered as a positive study for the corresponding indication(s).

Sample size calculations are based on 90% power and a type I error rate α = 0.05/4 = 0.0125.

Based on the FDA's document 'Summary Basis for Approval: Renova' (1993), the primary end point in the Renova studies (Ra) is highly correlated with Rz, a measurement of positive and negative light deflection used in several published studies of HRT. Given that there is no reliable Ra treatment effect estimate for HRT, the sample size is calculated based on the Rz measurement from Schmidt (1996). If we assume the difference of Rz-D measurements between the 1/10 and placebo groups is 9 µm and the corresponding standard deviation is 18 µm, the sample size for each group will be 121. Given a 30% dropout rate, the estimated sample size is 170 for each group.

The primary endpoint for investigator's assessment is defined as the percentage of subjects who experience a lack of deterioration. Deterioration is defined prospectively based on the placebo reference from the study.

Deterioration will be determined by taking the highest score such that at least 50% of placebo subjects have that change or greater.

Assume 50% of subjects have "lack of deterioration" in the placebo group, while in the 1/10 group, 75% do. The sample size is 113 based on α = 0.05/4 = 0.0125 and power = 0.90. Given a 30% dropout rate, the estimated sample size is 170 for each group.

Overall, a sample size of 170 per group seems to be sufficient, for a total of 510 subjects in the study.

Anticipated total number of subjects enrolled in the study will range from 400 to 600 subjects.

Studies examining the effect of continuous oral dosing of low dose NA and EE in over 1000 postmenopausal women demonstrate significant dose-related reductions in hot flash frequency and severity, as well as significant dose-related increases in bone mineral density, without deleterious effects on the endometrium. The associated adverse events reported with the combinations were relatively infrequent and as expected for clinical trials of HRT. The present study will evaluate the effects of 2 dosage combinations of NA and EE (1 mg NA/5 µg EE and 1 mg NA/10 µg EE) compared with placebo on skin aging in menopausal women.

The objectives of this study are to assess the effects of 2 dosage combinations of NA and EE-1 mg NA/5 µg EE and 1 mg NA/10 µg EE-compared to placebo in the control of skin wrinkling, dryness, and laxity associated with aging in menopausal women.

This is a randomized, double-blind, double-dummy, placebo-controlled multicenter study. Women who appear eligible for the study will be evaluated during a screening/baseline phase. Qualified women will be entered in the 48-week double-blind phase of the study and be randomly assigned to either 1 mg NA/5 µg EE, 1 mg NA/10 µg EE, or placebo. Those subjects in the 1/5 treatment group will receive active 1/5 tablets and 1/10 matching placebo tablets. Those in the 1/10 group will receive active 1/10 tablets and 1/5 matching placebo tablets. Those in the placebo group will receive both the 1/10 matching and 1/5 matching placebo tablets.

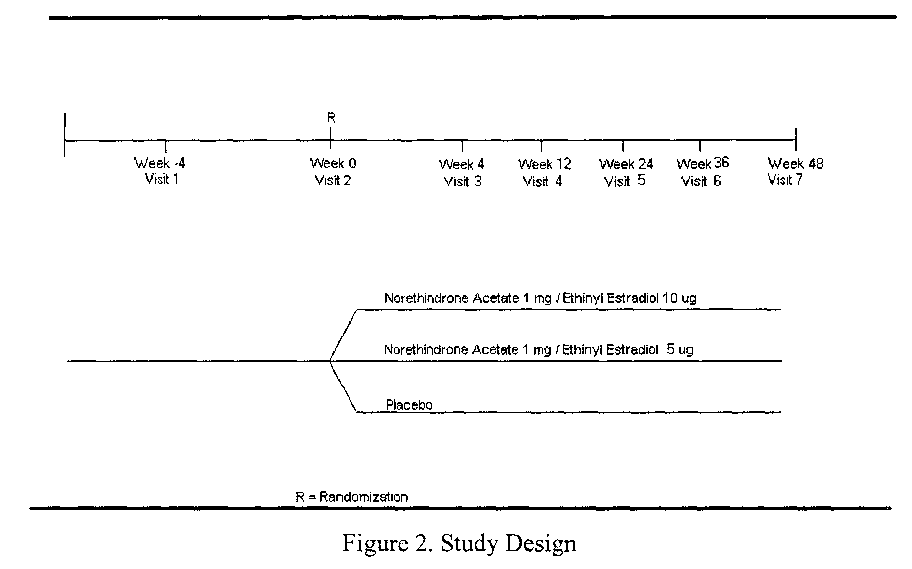

Subjects qualifying for the study will have the following procedures performed during the baseline phase (prior to randomization): Cutaneous replicas of the periorbital (crow's feet) and jowl areas, and MENQOL questionnaire, including supplemental modules covering skin and hair specific issues, treatment satisfaction, and additional items not covered in the original MENQOL.

In addition, at selected study centers, skin elasticity measurements of the jowl and upper cheek areas using the DermaLab® suction cup, and facial photographs.

Subjects who meet all inclusion criteria based on medical history, skin evaluation, physical and pelvic examinations, Pap smear, mammography, and routine laboratory tests including a negative pregnancy (hCG) test and do not violate any exclusion criteria may enter the treatment phase. Subjects who do not meet these requirements may not be rescreened at a later date.

In the treatment phase, subjects will be randomized as described below. Qualifying subjects will visit the clinic at the end of the screening/baseline phase and be randomized to 1 of 3 treatment groups according to a prepared randomization schedule. The treatment groups are: 1 mg NA/10 µg EE; 1 mg NA/5 µg EE; and Placebo.

Two bottles will be dispensed to each subject in each group with instructions to take 1 tablet from each bottle daily, in the evening, throughout the treatment phase of the study. Those subjects in the 1/10 treatment group will receive active 1/10 tablets and 1/5 matching placebo tablets. Those in the 1/5 group will receive active 1/5 tablets and 1/10 matching placebo tablets. Those in the placebo group will receive both the 1/10 matching and 1/5 matching placebo tablets. Calcium supplements will also be provided.

Subjects will be given daily subject diaries and instructed on their use. Women in early menopause who may be at risk for pregnancy should be advised to use an alternate means of contraception such as condoms or female barrier methods.

Subjects will return to the clinic at the end of Weeks 4, 12, 24, 36, and 48. At each visit the subject will have the following procedures performed:
- Clinical evaluation including blood pressure;
- Collection and review of subject daily diaries and medication compliance;
- Study medication will be dispensed and used medication containers collected; and
- Evaluation of concurrent medication use and query for adverse events.

In addition, at Weeks 12, 24, and 48:
- Investigator assessments of subject skin condition;
- Cutaneous replicas of the periorbital (crow's feet) and jowl areas; and
- At selected centers, skin elasticity measurements of the jowl and upper cheek areas using the DermaLab suction cup.

Additional procedures at Weeks 24 and 48:
- MENQOL, including supplemental modules covering skin and hair specific issues, treatment satisfaction, and additional items not covered in the original MENQOL.

At selected study centers, subject photographs will be obtained at Weeks 24 and 48.

At the final visit at Week 48, all subjects should have complete physical and pelvic examinations, a Pap smear, a TVU, a mammogram, and weight measured. Fasting blood and urine samples will be collected for routine safety measurements. If clinically indicated, an endometrial biopsy may be obtained.

A total of 510 subjects will be randomized. This study will utilize competitive enrollment. When this number of subjects is randomized to the double-blind phase of the study, the enrollment period will be closed.

Efficacy will be assessed by the measurement of primary and secondary efficacy parameters.

HRT (CI-376, femhrt) is a combination tablet containing NA and EE. For each active treatment group, each active tablet contains either 1 mg NA and 10 µg EE or 1 mg NA and 5 µg EE.

The dosage regimen is as described. Qualifying subjects will be given 2 bottles of study medication, Bottle A and Bottle B, and calcium tablets at the randomization visit (Visit 2, Study Day 1), 4 bottles of study medication at Visit 3 and 6 bottles at Visits 4, 5, and 6. Subjects will be instructed by the study coordinator or investigator to take 1 tablet from Bottle A and 1 tablet from Bottle B once a day in the evening and to take 2 tablets of calcium (1000 mg) each day. Subjects will be instructed to take the medication for the 48 weeks of the double-blind phase of the study.

As the Supplemental QOL modules are currently developmental, a full psychometric validation will be conducted in a validation study separate from this trial. This analysis will include assessment of instrument validity within the treatment population, homogeneity, discriminate validity, item internal consistency, evaluation of any floor or ceiling effects, scale-scale correlation, and clinical validity. The scoring algorithm for the supplemental modules will also be developed during the validation study. The Inferential Analysis Plan will be updated to reflect the results of the validation study. This will be done prior to unblinded data analysis. All QOL efficacy comparisons will be developed in detail in the Inferential Analysis Plan. Statistical power and sample size considerations are described below.

Sample size calculations are based on 90% power.

The primary efficacy parameter Ra is highly correlated with Rz based on the FDA's document 'Summary Basis for Approval: Renova' (1993). Given that there is no reliable Ra treatment effect estimate, the sample size is calculated based on the Rz measurement from Schmidt. If we assume the difference of Rz-D measurements between the 1/10 and placebo groups is 9 µm and the corresponding standard deviation is 18 µm, the sample size for each group will be 121. Given a 30% dropout rate, the estimated sample size is 170 for each group.

Assume that in the placebo group, 50% of subjects have lack of deterioration, while in the 1/10 group, 75% do. The sample size is 113 based on α = 0.05/4 = 0.0125 and power = 0.90. Given a 30% dropout rate, the estimated sample size is 170 for each group.

Overall, a sample size of 170 per group seems to be sufficient, for a total of 510 subjects in the study.

### Glossary

- Fine wrinkling:: This factor represents a visual assessment of the number and depth of superficial wrinkles (i.e., shallow indentations or lines). Fine wrinkles typically appear in periorbital and perioral regions and are usually found further from the eyes and mouth than are coarse wrinkles.
- Coarse wrinkling:: This factor represents a visual assessment of the number and depth of coarse wrinkles (i.e., deep lines, furrows, or creases). Coarse wrinkles appear on the forehead, glabella, chin, and nasolabial and periorbital areas, and they tend to be located closer to the eyes and mouth than fine wrinkles.
- Ra:: Replica analysis that determines the area of deviation (AUC) above and below a central line.
- Rz:: Replica analysis that determines the average difference between the maximum and minimum heights.
- Shadows:: Replica analysis that determines the total area within a standard field that is occupied by dark shadows.

Facial skin is evaluated by the investigator according to the following protocol.

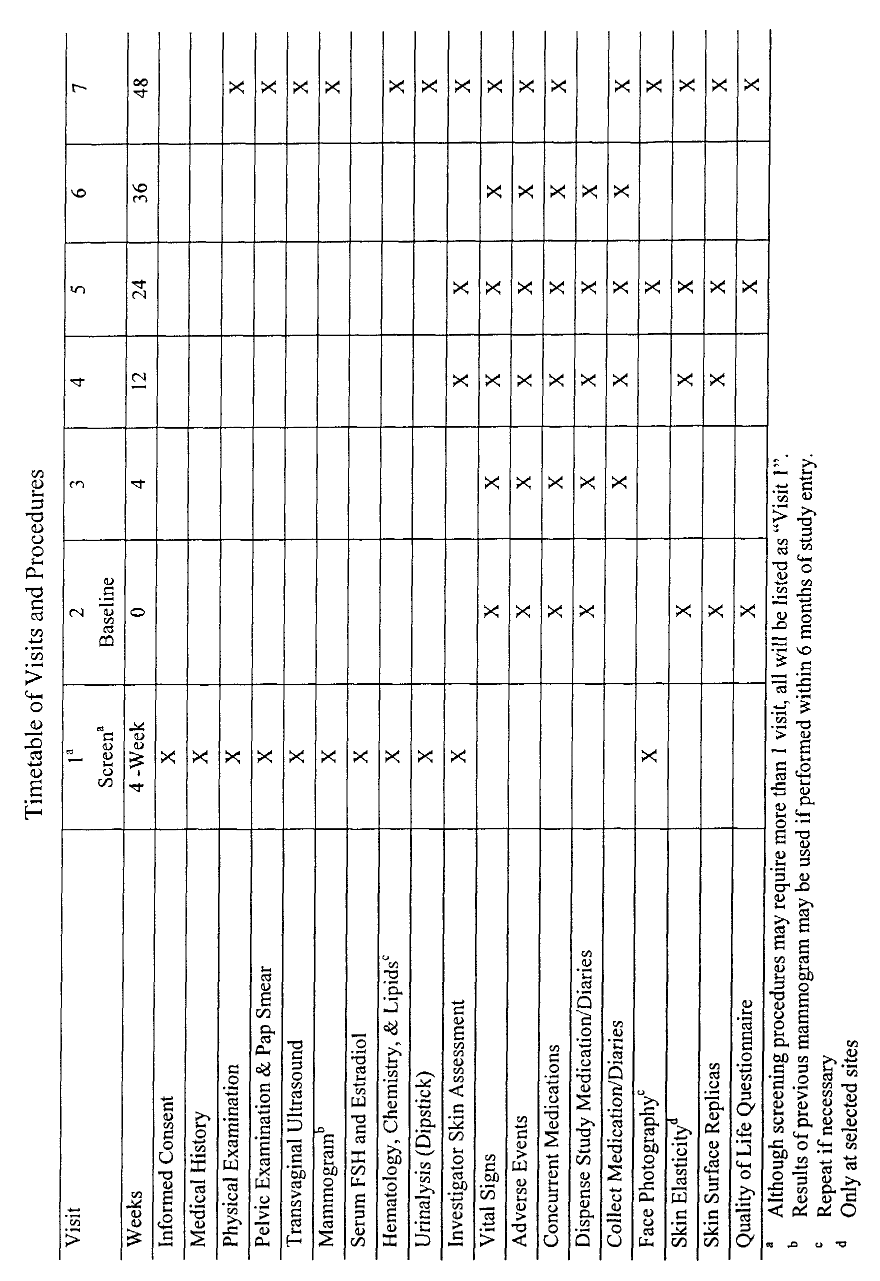

Currently there are no photonumeric or descriptive scales to assess intrinsic skin aging. Several scales have been developed to assess extrinsic photoaging. A 10-point scale was used by Griffiths C, Wang TS, Hamilton TA, Voorhees JJ, Ellis CN. A photonumeric scale for the assessment of cutaneous photodamage. *Arch Dermatol*., 1992;128:347-351, to evaluate cutaneous photodamage. Using this example, scales were developed to evaluate skin wrinkling, laxity, and texture.

The photonumeric and descriptive scales will be provided as guides for the investigator in evaluation of the degree of skin changes associated with aging. Components of skin aging will be separated into 10 grades (0-9). The scales will range from the following:
(0: None; 1-3: Mild; 4-6: Moderate; 7-9: Severe)

Degree of change must be mild to moderate in order to qualify for this study. Any subject with a rating of 7 or higher in any area will be excluded.
Facial skin wrinkling and laxity is assessed.

Facial photographs of postmenopausal women were evaluated and selected as representative examples of skin aging. Groups of photographs demonstrating increasing severity of skin aging (wrinkling or laxity/sagging) in each of the facial areas listed below were assigned grades of: 0 absent, 1 to 3 mild, 4 to 6 moderate, or 7 to 9 severe. Photographs were assigned grades for: Wrinkling of the periorbital area (crow's feet), perioral area, and of the suborbital area is evaluated; and laxity/sagging of suborbital area and of the jowl area is measured according to the following protocol.

Using the photonumeric reference booklet as a guide, the investigator should select a score of 0 to 9 for each of the above areas after visual examination of the subject. The score should be entered on the appropriate CRF. Investigator evaluations of a subject should be done by the same investigator throughout the study.
Global assessment of facial skin wrinkling is assessed.

The investigator should obtain a global facial wrinkling score by adding the scores for wrinkling of the periorbital, perioral, and suborbital areas. This score should be divided by 3 and entered on the CRF with the individual area scores. Global wrinkling then is absent for a score of 0, mild 1 to 3, moderate 4 to 6, and severe 7 to 9.
Global assessment of facial skin laxity/sagging is scored.

The investigator should obtain a global facial laxity/sagging score by adding the scores for laxity/sagging of the suborbital and jowl areas. This score should be divided by 2 and entered on the CRF with the individual area scores. Global laxity/sagging then is absent for a score of 0, mild 1 to 3, moderate 4 to 6, and severe 7 to 9.
Global assessment of facial skin texture/dryness is rated.

This investigator should select a score after visual and tactile examination of the forehead and cheeks of the subject. The score should be entered on the appropriate CRF.

| Score | Description |
|---|---|
| 0 Absent | No visible signs of uplifted scales; skin is smooth to touch |
| 1-3 Mild | No visible signs of uplifted scales; mild roughness to touch |
| 4-6 Moderate | Some area of visible scales; moderate roughness to touch |
| 7-9 Severe | Many areas of visible scaling; very rough to the touch |

### Procedure for Skin Replicas

### Skin Surface Impression Technique Using Silflo Materials

1. Loading the Syringe: When opening a new bottle of Silflo, empty the bottle of thinner into it and mix well, stirring with a spatula. Shaking is difficult because of the viscosity. Then pour the Silflo into a 10-cc syringe. Remember to empty the contents of the syringe back into the bottle at the end of the work day because the mixture tends to separate in the syringe as well as the bottle.
2. Preparing the Subject: Make sure the subject has washed the area about 30 minutes prior to the replica. Oils and make-up prevent good results. Place a labeled location ring (can be obtained from CuDerm Corp in Dallas) on the area of interest. Make sure there is no tension on the skin. Also make sure that the tab on the ring is pointed in the same direction each time. When doing an impression of the crow's feet, you do not want the subject lying because this creates a facelift effect. Make sure to take a close-up photo of the ring in place at baseline so you have a reference for the following visits. After the ring is in place, then the subject can lie down to make it easier for the technician to apply the Silflo.
3. Preparing the Material: For one replica, place 0.4 cc (2 divisions on the syringe) of Silflo onto a piece of wax paper. Place one drop of catalyst onto the Silflo and immediately mix. You need to make sure that the one drop of catalyst is mixed with all of the Silflo. After about 20 seconds, begin to gather the material onto the spatula and spread it over the hole with swiping motions. Replace the lid on the catalyst because it does have a shelf life and will dry and seal the hole. If the hole does dry up, use a pin or paper clip to open it. This is also necessary when you open a new bottle.
4. The Impression: After a couple minutes, check for dryness by touching the back of the replica. If it is not tacky, pull off, remove the tape, and place into a labeled glassine envelope. The ring should also be labeled. When beginning, you may want to do 2 replicas at each site so the Skin Center can choose the best one, based on technique. If there is still white material on the skin under the hole after the ring is removed, the material was not completely mixed. Shiny spots will also appear on the replica. Please do another one.

At the Skin Study Center, image analysis will be performed. Each replica will be placed under a camera attached to a computer. A light is shone at approximately 25 degrees onto the replica. This causes shadows to be produced onto the negative replica of the skin. The digital image is captured and then analyzed using a program written in the Image Pro Plus software package. The percent area covered by the shadows (SH) is calculated. The lower the number, the smoother the skin.

### Skin Elasticity

### Cortex Technology DermaLab® Suction Cup

With aging, the skin does not behave as an ideal object, and with continuing stress the deformation increases slowly. This behavior is termed "creep," and it is a consequence of the viscous extension of the skin. When stress is relieved, the skin does not immediately return to its original state, but remains slightly deformed, a phenomenon known as hysteresis, which is also a consequence of viscous properties.

A DermaLab® with an Elasticity Module will be used to evaluate skin elasticity. The suction probe which is placed on the test site is capable of producing a vacuum up to 65 kPa and consists of an upper and lower sensor with a 1.5 mm elevation. The measuring aperture is 10 mm in diameter, and the probe itself has an ultra low weight of approximately 7 g for minimum skin bias. The probe is secured to the panelist's site using an adhesive ring. Once activated, the vacuum is applied, and once the skin reaches the lower sensor, the measurement begins and continues until the skin reaches the upper sensor. The skin is then allowed to relax for 10 seconds before the vacuum resumes for a total of 4 cycles. E is the ratio based on the pressure differential observed at the lower and upper sensors and is equivalent to the elastic modulus.

Using the DermaLab Suction Cup:
1. Make sure your DermaLab is on and on the correct channel.
2. Have the area to be measured in a comfortable position.
3. Remove the adhesive ring from the roll.
4. Peel off paper backing.
5. Placing the probe between your thumb and forefinger, center the adhesive ring on the probe making sure not to overlap the measurement aperture.
6. Firmly place the probe on the surface to be measured. If this is a vertical surface, steps should be taken to make sure the probe is not being weighted down, such as holding the cord.
7. Press the start button and wait for the measurement to finish.
8. The printout report is considered as the source document and should be filed with the subject's chart. Complete the appropriate CRF.

### Procedure for Facial Photography

### Photographic Procedure

In these clinical photographs, for the duration of the study, the only thing allowed to change is the skin condition itself. Therefore, anything extraneous to the condition (jewelry, makeup, clothing, furniture, walls, etc) is to be eliminated from the fields to be photographed, from the screening through the final photographs. The necessity of good end-of-study photos should be stressed to the subjects to ensure their cooperation. Film emulsion, lighting, framing, exposure, and reproduction ratios must be held constant. In the end, the pictures should read like a time-lapse movie.

### EXAMPLE 2

### Efficacy and Safety of Estrostep in the Treatment of Moderate Acne Vulgaris―A 6-Month Randomized, Double-Blind, Placebo-Controlled, Parallel Group Multicenter Study

This study was designed to assess the efficacy and safety of Estrostep compared with placebo in the treatment of moderate acne vulgaris for 6 months.

**Methodology:** A 6-cycle, randomized, double-blind, placebo-controlled, parallel group, multicenter study assessing the efficacy and safety of Estrostep in the treatment of moderate acne vulgaris in normally cycling woman.

**Diagnosis and Criteria for Inclusion:** Healthy female subjects, aged 14-49, ≥1 year postmenarche, baseline menstrual cycle ≤42 days, moderate facial acne with 20 to 100 comedones and 20 to 65 inflammatory lesions (papules or pustules) and no more than 5 nodules, have not responded adequately to topical antiacne therapy. Subjects who were pregnant or nursing, had other significant facial disease or concomitant systemic disease, or who had evidence of significant endocrinopathy such as marked hirsutism were excluded from the study.

### Primary Efficacy Endpoints:

- Lesion counts: Change from baseline to study exit in total number of acne lesions, inflammatory lesions and comedones
- Facial Acne Global Assessment at study exit

### Statistical Methods:

- Lesion Counts:
   Differences between treatment groups with respect to the change from baseline to study exit were analyzed using an analysis of covariance (ANCOVA) model including effects for treatment group, baseline lesion count, and investigator. Ninety-five percent confidence intervals about the model estimated treatment effect (Estrostep - placebo) were calculated.
- Facial Acne Global Assessment
   Facial Acne Global Assessments for the treatment groups were compared at study exit using Cochran-Mantel-Haenzel (CMH) methodology stratified by investigator. For the primary analysis, ratings of "absent," "minimal," and "mild" were considered "improved"; other ratings were considered "not improved." For the supportive analysis, ratings of "absent" or "minimal" were considered "improved"; other rating were considered "not improved."
   The primary inference is based on the intention-to-treat (ITT) population, with similar analyses performed on an "evaluable" population, which was defined on a blinded basis.

### Secondary and Safety Analyses:

- Lesion Counts at each visit
- Testosterone, SHBG, free testosterone, DHEAS
- Patient Assessment of Acne Severity
- Acne Specific Quality of Life
- Adverse Events (all, associated and by intensity), Serious Adverse Events, Withdrawls due to Adverse Events, Deaths
- Clinically significant variations in laboratory parameters

Two study groups were employed:

### Populations:

In one study group (Group 1) 298 women were randomized at 17 US centers in the United States (US). The primary population for analysis was specified to be the ITT population.
- ITT population: 298 women were included in the ITT population, approximately two-thirds were caucasian with a median age of 23 years and 22% were under the age of 18. The median time since onset of acne was about 8 years).
   - Lesion Counts: All 298 women were included in the ITT analysis of lesion counts.
   - Facial Acne Global Assessment: The study was started using a 5 point scale for this endpoint. At the FDA's request, the scale was changed to a 7 point scale. A total of 20 patients (10 in each group) were excluded from the ITT analysis of the Facial Acne Global Assessment, because a 7 point scale was not administered.
- Evaluable population: 221 of 298 subjects completed at least the first 3 cycles of treatment, were compliant with study medication and had no other significant protocol violations (104 randomized to placebo, 117 to Estrostep). The difference between the ITT and evaluable populations was primarily due to patients being withdrawn prior to Cycle 3 (23 randomized to placebo, 16 to Estrostep). In addition, 19 patients were planned early terminations resulting from the decision to close the study early for administrative reasons. These patients generally received about 4-5 cycles of treatment and were included in the evaluable population.

In the second study group (Group 2), 293 women were randomized at 18 centers in the US. The primary population for analysis was specified to be the ITT population.
- ITT population: 293 women were included in the ITT population, approximately two-thirds were caucasian with a median age of 23 and 24% under the age of 18. The median time since onset of acne was about 8 years.
   - Lesion counts: All 293 women were included in the ITT analysis of lesion counts.
   - Facial Acne Global Assessment: A total of 16 patients (10 patients randomized to placebo, 6 to Estrostep) are excluded from the ITT analysis of the, because a 7 point scale was not administered.
- Evaluable population: 222 of 293 subjects completed at least the first 3 cycles of treatment, were compliant with study medication and had no other significant protocol violations (112 randomized to placebo, 110 to Estrostep.

### Demographics

There were no differences between the 2 studies in terms of demographics. The mean age was 24 (range; 13-48) with 22% of the population under age 18. Two-thirds of the subjects were caucasian and one-third were from minority groups; primarily black, Hispanic, and Asian. One quarter of the subjects were past or current smokers.

### Results―Lesion Counts

**TABLE 2**

| Lesion Count Change From Baseline to Study Exit―Group 1 | | | | | |
|---|---|---|---|---|---|
| | Placebo N=148 | Estrostep N=150 | Treatment Effect^{a} | 95% C.I. | p-value^{b} |
| **Total Lesion Count** | | | | | |
| Baseline | | | | | |
| Mean (SD) | 75.3 (30.4) | 77.0 (26.5) | | | |
| Change from Baseline | | | | | |
| **LS Mean** | **-24.2** | **-30.9** | **-6.6** | **(-12.5, -0.8)** | **0.0279** |

| **Inflammatory Lesion Count** | | | | | |
|---|---|---|---|---|---|
| Baseline | | | | | |
| Mean (SD) | 29.7 (10.5) | 29.3 (10.5) | | | |
| Change from Baseline | | | | | |
| **LS Mean** | **-12.5** | **-14.7** | **-2.2** | **(-4.6, 0.2)** | **0.0747** |

| **Total Comedone Count** | | | | | |
|---|---|---|---|---|---|
| Baseline | | | | | |
| Mean (SD) | 45.6 (25.3) | 47.7 (22.9) | | | |
| Change from Baseline | | | | | |
| **LS Mean** | **-11.9** | **-16.3** | **-4.5** | **(-8.9, -0.1)** | **0.0466** |

| | | | | | |
|---|---|---|---|---|---|
| ^{a} Model Based Treatment Effect: Treatment effect=Estrostep-placebo | | | | | |
| ^{b} ANCOVA F Test: includes: treatment, baseline lesion count, and investigator Notes: 1) Met FDA Criteria with ITT Analyses (2 out of 3 are significant). 2) None of the analyses of the evaluable population were significant (p = 0.1731, 0.3393, and 0.230). This is due to slightly reduced estimates of treatment effect (although all go in the proper direction) and the reduced sample size. | | | | | |

**TABLE 3**

| Lesion Count Change From Baseline to Study Exit―Group 2 | | | | | |
|---|---|---|---|---|---|
| | Placebo N=147 | Estrostep N=146 | Treatment Effect^{a} | 95% C.I. | p-value^{b} |
| **Total Lesion Count** | | | | | |
| Baseline | | | | | |
| Mean (SD) | 69.2 (24.4) | 70.2 (25.0) | | | |
| Change from Baseline | | | | | |
| **LS Mean** | **-24.0 (24.0)** | **-33.7 (24.5)** | **-8.2** | **(-12.9, -3.4)** | **0.0008** |

| **Inflammatory Lesion Count** | | | | | |
|---|---|---|---|---|---|
| Baseline | | | | | |
| Mean (SD) | 29.2 (10.1) | 29.7 (8.7) | | | |
| Change from Baseline | | | | | |
| **LS Mean** | **-12.5 (12.3)** | **-15.4 (12.1)** | **-2.9** | **(-5.3, -0.5)** | **0.0177** |

| **Total Comedone Count** | | | | | |
|---|---|---|---|---|---|
| Baseline | | | | | |
| Mean (SD) | 40.0 (19.7) | 40.6 (21.9) | | | |
| Change from Baseline | | | | | |
| **LS Mean** | **-9.7 (18.6)** | **-14.9 (-5.2)** | **-5.2** | **(-8.6, -1.8)** | **0.0031** |

| | | | | | |
|---|---|---|---|---|---|
| ^{a} Model Based Treatment Effect: Treatment effect=Estrostep-placebo | | | | | |
| ^{b} ANCOVA F Test: includes: treatment, baseline lesion count, and investigator Notes: 1) Results are strong and consistent. Additional analyses have been defined for the ISE, to test whether results are statistically different across the two studies. If so, differences between studies may be explored systematically (more likely that differences are primarily due to chance; note wide confidence intervals). 2) Analyses of evaluable population are highly significant (stronger than ITT analyses). 3) Results are consistent with Lesion Count reductions seen in the Ortho TriCyclen® (Ortho-McNeil) studies (Table 4). | | | | | |

**TABLE 4**

| Comparison of Estrostep and Ortho-TriCyclen® (Ortho-McNeil) Data With Lesion Counts; Data is Reduction in Lesions Between Treatment and Placebo (Bold represents statistically significant reductions) | | | | |
|---|---|---|---|---|
| Lesion Type | Ortho® (Ortho-McNeil) -034 (n = 227) | Ortho® (Ortho-McNeil) -035 (n = 228) | Group 1 (n = 298) | Group 2 (n = 293) |
| Total | 4.5 | 11.4 | 6.6 | 8.2 |
| Inflammatory | 2.7 | 3.9 | 2.2 | 2.9 |
| Comedones | 1.7 | 7.5 | 4.5 | 5.2 |

### Results―Facial Acne Global Assessment

**TABLE 5**

| Facial Acne Global Assessment at Study Exit [Number (%) of Subjects] Primary Analysis Intent-to-Treat Population―Study Group 1 | | | |
|---|---|---|---|
| Assessment^{a} | Placebo N = 138 | Estrostep N = 140 | p-value^{a} |
| Primary Analysis | | | |
| Absent + Minimal + Mild | 42 (30%) | 56 (40%) | 0.1435 |
| Mild to Moderate or Worse | 96 (70%) | 84 (60%) | |

| Secondary Analysis (suggested by FDA) | | | |
|---|---|---|---|
| Absent + Minimal | 10 (7%) | 23 (16%) | 0.0023 |
| Mild or worse | 128 (93%) | 117 (84%) | |

| | | | |
|---|---|---|---|
| ^{a} Cochran-Mantel-Haenzel chi-square test of Improved vs Not Improved by comparing treatment group stratified by investigator | | | |

**TABLE 6**

| Facial Acne Global Assessment at Study Exit [Number (%) of Subjects] Summary by Category Intent-to-Treat Population Group 1 | | |
|---|---|---|
| Assessment^{a} | Placebo N = 138 | Estrostep N = 140 |
| Absent | 0 (0%) | 2 (1%) |
| Minimal | 10 (7%) | 21 (15%) |
| Mild | 32 (23%) | 33 (24%) |
| Mild to Moderate | 49 (36%) | 51 (36%) |
| Moderate | 40 (29%) | 27 (19%) |
| Marked | 7 (5%) | 5 (4%) |
| Severe | 0 (0%) | 1(1%) |

| | | |
|---|---|---|
| ^{a} Cochran-Mantel-Haenzel chi-square test of Improved vs Not Improved by comparing treatment group stratified by investigator Notes: 1) Secondary analysis using criteria suggested by FDA of improved = minimal + absent is statistically significant (7% vs 16%, p-value = 0.0223). 2) Primary analysis is not statistically significant and analysis of evaluable population is similarly not significant (p-value = 0.3199). 3) Lesion count by study visit indicate a greater decrease in all lesion counts for the Estrostep treated subjects beginning at Cycle 3 and continuing for the duration of the study (Figures C.1, C.2, C.3). | | |

**TABLE 7**

| Facial Acne Global Assessment at Study Exit [Number (%) of Subjects] Primary Analysis Intent to Treat Population Group 2 | | | |
|---|---|---|---|
| Assessment^{a} | Placebo N = 137 | Estrostep N = 140 | p-value^{b} |
| Primary Analysis | | | |
| Absent + Minimal + Mild | 40 (29%) | 68 (49%) | 0.0006 |
| Mild to Moderate or Worse | 97 (71 %) | 72 (51 %) | |

| Secondary Analysis (suggested by FDA) | | | |
|---|---|---|---|
| Absent + Minimal | 11 (8%) | 24 (17%) | 0.0174 |
| Mild or Worse | 126 (92%) | 116 (83%) | |

| | | | |
|---|---|---|---|
| ^{a} Responses of "absent," "minimal," or "mild" are considered "improved"; other responses are considered "not improved." | | | |
| ^{b} Cochran-Mantel-Haenzel chi-square test of Improved vs Not Improved by comparing treatment group stratified by investigator | | | |

**TABLE 8**

| Facial Acne Global Assessment at Study Exit [Number (%) of Subjects] Summary by Category Intent-to-Treat Population Group 1 | | |
|---|---|---|
| Assessment^{a} | Placebo N = 137 | Estrostep N = 140 |
| Absent | 0 (0%) | 0 (0%) |
| Minimal | 11 (8%) | 24 (17%) |
| Mild | 29 (21 %) | 44 (31 %) |
| Mild to Moderate | 49 (36%) | 40 (29%) |
| Moderate | 31 (23%) | 27 (19%) |
| Marked | 13 (9%) | 5 (4%) |
| Severe | 4 (3%) | 0 (0%) |

| | | |
|---|---|---|
| ^{a} Responses of "absent," "minimal," or "mild" are considered "improved"; other responses are considered "not improved." Notes: 1) All analyses are statistically significant (including FDA suggested secondary analysis that was significant in Group 1 and analyses of evaluable population). 2) Lesion count by study visit indicate a greater decrease in all lesion counts for the Estrostep treated subjects beginning at cycle 3 and continuing for the duration of the study (Figures C.1, C.2, C.3). | | |

**TABLE 9**

| Facial Acne Global Assessment: P values for the Comparison of the Distributions of Ratings Favoring Estrostep by Visit (Intent-to-Treat Population) | | |
|---|---|---|
| | Group 1 | Group 2 |
| Cycle (Visit) | P value | P value |
| Cycle 1 (V-3) | 0.7199 | 0.4988 |
| Cycle 2 (V-4) | 0.8556 | 0.7688 |
| Cycle 3 (V-5) | 0.4602 | 0.1254 |
| Cycle 4 (V-6) | 0.0040 | 0.0100 |
| Cylce 5 (V-7) | 0.0387 | 0.0038 |
| Cycle 6 (V-8) | 0.0089 | 0.0003 |
| Cycle 6 (V-9) | 0.0333 | 0.0017 |
| Cycle 6 (V-10) | 0.1065 | 0.0020 |

### Results―Androgen Levels

There was a 20% reduction in total testosterone, a 2-3 fold increase in SHBG, resulting in a 60% to 70% decrease in free testosterone. There was also a decline in the adrenal androgen, DHEA-S.

### Results―Patient Assessment of Acne Severity and

### Acne Specific Quality of Life

The patient assessment of acne severity, modeled on the tool used by Ortho TriCyclen® (Ortho-McNeil), was positive in favor of Estrostep.

The Acne-specific Quality of Life questionnaire (Acne-QoL) was developed and validated in male and female subjects (13-35 years). The instrument is organized into four domains (self perception, role-emotional, role-social and acne symptoms) which address the impact of facial acne on health-related quality of life.

ANCOVA was conducted to evaluate changes from Baseline to Cycle 3 (Visit 5) and Baseline to Cycle 6 (Final Visit) in each of the four domains of the Acne-QoL. Estrostep demonstrated a statistically significant advantage over placebo at both timepoints for all four domains.

**TABLE 10**

| Acne-specific Quality of Life Evaluation-Intent to Treat Population Group 1/Group 2 Pooled Analysis | | | | | |
|---|---|---|---|---|---|
| | Placebo N = 295 | Estrostep N = 296 | Treatment Effect^{a} | 95% CI | p-value^{b} |
| **Self Perception** | | | | | |
| Baseline Mean (SD) | 19.97 (7.48) | 20.12 (7.93) | | | |
| Change from Baseline to Cycle 3 ^{c} | 2.29 (7.41) | 3.46 (7.22) | 1.17 | 0.07, 2.27 | 0.0384 |
| Change from Baseline to Cycle 6 ^{c} | 2.84 (8.12) | 6.24 (8.62) | 3.40 | 2.24, 4.57 | <0.0001 |
| | | | | | |

| **Role-Emotional** | | | | | |
|---|---|---|---|---|---|
| Baseline Mean (SD) | 19.19 (7.48) | 18.77 (7.85) | | | |
| Change from Baseline to Cycle 3 ^{c} | 1.97 (7.03) | 3.47 (7.45) | 1.50 | 0.39, 2.60 | 0.0080 |
| Change from Baseline to Cycle 6 ^{c} | 2.94 (7.88) | 6.45 (9.02) | 3.50 | 2.30, 4.70 | <0.0001 |
| | | | | | |

| **Role-Social** | | | | | |
|---|---|---|---|---|---|
| Baseline Mean (SD) | 19.72 (6.67) | 19.48 (6.85) | | | |
| Change from Baseline to Cycle 3 ^{c} | 0.86 (5.16) | 2.08 (5.58) | 1.23 | 0.42, 2.03 | 0.0029 |
| Change from Baseline to Cycle 6 ^{c} | 1.40 (5.55) | 3.63 (6.48) | 2.23 | 1.40, 3.06 | <0.0001 |
| | | | | | |

| **Acne Symptoms** | | | | | |
|---|---|---|---|---|---|
| Baseline Mean (SD) | 19.08 (5.30) | 18.75 (5.34) | | | |
| Change from Baseline to Cycle 3 ^{c} | 2.11 (5.52) | 3.48 (5.46) | 1.37 | 0.56, 2.19 | 0.0011 |
| Change from Baseline to Cycle 6 ^{c} | 3.12 (5.90) | 5.63 (5.89) | 2.50 | 1.64, 3.36 | <0.0001 |

| | | | | | |
|---|---|---|---|---|---|
| ^{a} Treatment effect = Estrostep-placebo | | | | | |
| ^{b} ANCOVA includes treatment, baseline measure and investigator | | | | | |
| ^{c} Analyses were performed on LS Means Note: Each of the 4 domains consists of a set of questions; the answers are summed for an overall domain score. A high score represents a more favorable quality of life. | | | | | |

### Safety

There are no safety issues. There were no deaths. There was one SAE that was not associated with treatment. Other adverse events were primarily mild or moderate and were as expected for oral contraceptive use. There were 13 pregnancies, all but 1 occurred in the placebo group.

| Adverse Events | | | | |
|---|---|---|---|---|
| | Group 1 | | Group 2 | |
| | Placebo | Estrostep | Placebo | Estrostep |
| All AEs | 51 (34%) | 74 (49%) | 92 (62%) | 102 (69%) |
| Associated AEs | 19 (13%) | 39 (26%) | 28 (19%) | 59 (40%) |
| Mild AEs | 18 (35%) | 31 (42%) | 37 (40%) | 45 (44%) |
| Moderate AEs | 31 (61%) | 35 (47%) | 41 (45%) | 46 (45%) |
| Severe AEs | 0 (0%) | 8 (11%) | 11 (12%) | 10 (10%) |
| Serious AEs | 0 (0%) | 1 (1%)* | 0 (0%) | 0 (0%) |
| Withdrawals | 8 (5%) | 13 (9%) | 7 (5%) | 8 (5%) |

| | | | | |
|---|---|---|---|---|
| * Not associated with Drug Treatment | | | | |

| Associated Adverse Events | | | | |
|---|---|---|---|---|
| | Group 1 | | Group 2 | |
| | Placebo | Estrostep | Placebo | Estrostep |
| Migraine | 0 (0%) | 6 (4%) | 11 (7%) | 7 (5%) |
| Nausea | 2 (1%) | 11 (7%) | 4 (3%) | 5 (3%) |
| Metrorrhagia | 2 (1% | 14 (9%) | 5 (3%) | 37 (25%) |

### Conclusions

- In the Study Group 2, patients clearly meets all endpoints, and analyses are confirmed with secondary analyses.
- In the Study Group 1, patients have met the criteria of 2 of the 3 count endpoints are statistically significant in the ITT population. This result is not confirmed statistically in the evaluable population, although the data is qualitatively similar in that population.
- The magnitude of the treatment effects are similar to those seen with Ortho-Tri-Cyclin® (Ortho-McNeil) (see Table 8, data from FOI). There is not comparable data on the global assessment, due to a use of a different assessment instrument in the Ortho-Tri-Cycline® (Ortho-McNeil) studies.
- There is not a statistically significant difference in the global assessment in Group 1 using the primarily defined cutoff; however, a secondary analysis using a cutoff suggested by FDA does show statistical significance. In addition, comparison of the distributions of ratings at each time point indicate a clear separation favoring Estrostep beginning at Cycle 4. There are no real differences between the populations or results in the two studies and combination of the results from both studies shows positive results on all lesion counts and the global assessment.
- The patient assessment of acne severity and the acne specific quality of life were strongly positive in favor of Estrostep.
- There are no safety issues. Associated Adverse Events are those expected with oral contraceptives.
- There were 13 pregnancies, all but 1 were in the placebo group.

## Claims

1. A method of preparing a medicament comprising an effective amount of an estrogen and a progestogen for use in delaying the onset and treating skin aging.

2. A method of preparing a medicament comprising an effective amount of a fixed quantity of a synthetic estrogen and a synthetic progestin for use in delaying the onset and treating skin aging.

3. A method of preparing a medicament comprising an effective amount of a fixed quantity of norethindrone acetate (NA) and ethinyl estradiol (EE) for use in delaying the onset and treating skin aging.

4. A method of preparing a medicament comprising an effective amount of compositions containing 1 mg NA and 5 µg EE for use in delaying the onset and treating skin aging.

5. A method of preparing a medicament comprising an effective amount of 1 mg NA and 10 µg EE in a pharmaceutically acceptable carrier for use in delaying the onset and treating skin aging.

6. A method of preparing a medicament comprising an effective amount of an estrogen and a progestogen for use in preventing and treating acne in a patient.

7. A method of preparing a medicament comprising an effective amount of a synthetic estrogen in combination with a synthetic progestin for use in the treatment and prevention of acne.

8. A method of preparing a medicament comprising an effective amount of norethindrone acetate in combination with ethinyl estradiol for use in treating and preventing acne.

9. A method of preparing a medicament comprising an effective amount of 1 mg norethindrone acetate and a gradually increasing dose of ethinyl estradiol; 20 mcg × 5 days, 30 mcg × 7 days, and 35 mcg × 9 days for use in preventing and treating acne.

10. A method according to Claim 9 wherein the acne treated is acne vulgaris or cystic acne.

11. A method of preparing a medicament comprising an effective amount of Estrostep for use in preventing and treating acne.

12. A method according to Claim 11 wherein the acne treated is acne vulgaris or cystic acne.
